# EUROPEAN PATENT SPECIFICATION

(11) **EP 1 359 881 B1**
(45) Date of publication and mention of the grant of the patent: **20.02.2008**
(21) Application number: 02715376.6
(22) Date of filing: 17.01.2002
(51) Int. Cl.: A61J 1/00, A61K 31/575

(54) **CONTAINER WITH LOW OXYGEN CONTENT CONTAINING FF-MAS**
BEHÄLTNIS MIT NIEDRIGEM SAUERSTOFFGEHALT, DAS FF-MAS ENTHÄLT
CONTENANT A FAIBLE TENEUR EN OXYGENE ET RENFERMANT UNE SUBSTANCE ACTIVANT LA MEIOSE

(30) Priority: 06.02.2001 DK 200100189; 08.03.2001 DK 200100382
(43) Date of publication of application: 12.11.2003
(73) Proprietor: NOVO NORDISK A/S, 2880 Bagsvaerd (DK)
(72) Inventor: MÜLLER, Lars, Klingberg, DK-2750 Ballerup (DK); ANDERSEN, Tina, Meinertz, DK-2970 Horsholm (DK)
(86) International application number: PCT/DK2002/000035
(87) International publication number: WO 2002/062287

(56) References cited:
- EP-A1- 0 709 304
- WO-A1-90/03429
- WO-A1-98/28323

## Description

The present invention relates to a solid, chemically stable composition that can be used in connection with in vitro fertilisation or in vitro maturation (hereinafter designated IVF or IVM, respectively). More specifically, it relates to a container containing such a solid composition, as defined in claim 1.

### BACKGROUND OF THIS INVENTION

Several meiosis activation substances, e.g. 4,4-dimethyl-5α-cholesta-8,1 4,24-triene-3β-ol, are known e.g. from WO-A-98/28323. When 4,4-dimethyl-5α-cholesta-8,14,24-triene-3β-ol is kept in a medium containing oocytes, the oocytes become more prone to fertilisation. However, a major problem with the use of 4,4-dimethyl-5α-cholesta-8,14,24-triene-3β-ol is that it has a very low solubility and low chemical stability under the conditions at which it is to be stored and used.

The object of this invention is to overcome or ameliorate at least some of the disadvantages of the prior art. Hence, not all the more detailed objects mentioned below may be fully overcome or ameliorated.

One object of this invention is to develop a composition containing 4,4-dimethyl-5α-cholesta-8,14,24-triene-3β-ol or a derivative thereof that can be dissolved in an aqueous medium in a sufficient concentration to be used for IVF or IVM.

Another object is to develop a composition containing 4,4-dimethyl-5a-cholesta-8,14,24-triene-3β-ol or a derivative thereof which can be dissolved in an aqueous medium without any physical influence such as heating, stirring, or ultrasound treatment.

A third object is to develop a composition containing 4,4-dimethyl-5α-cholesta-8,14,24-triene-3β-ol or a derivative thereof which has a sufficient chemical and physical stability at the conditions under which it is stored and used.

The solubility of 4,4-dimethyl-5α-cholesta-8,14,24-triene-3β-ol (herein designated FF-MAS), in water is very low, i.e., approximately 20 picogram/ml (corresponding to 2 x 10⁻⁵ µg/ml). In ethanol, the solubility of FF-MAS is substantially higher, i.e., approximately 12 mg/ml. According to our preliminary investigations, the highest solubility of FF-MAS in a mixture of ethanol and water (1:2.5) is approximately 0.4 mg/ml. Several other meiosis activating substances (herein designated MASs) have a similar low solubility in water.

### DETAILED DESCRIPTION OF THIS INVENTION

Surprisingly, it has been found that the stability of 4,4-dimethyl-5α-cholesta-8,14,24-triene-3β-ol can be improved when it is stored in the presence of only a minor amount of oxygen. Hence, one embodiment of this invention relates to a closed container having a low content of oxygen and further containing 4,4-dimethyl-5α-cholesta-8,14,24-triene-3β-ol. This invention relates to a closed container, wherein the low content of oxygen is below about 0.01 mole oxygen per litre container volume, preferably below about 0.001 mole oxygen per litre container volume, even more preferred below about 0.0001 mole oxygen per litre container volume.

Furthermore, surprisingly it has now been found that a solid composition containing 4,4-dimethyl-5α-cholesta-8,14,24-triene-3β-ol and an additive has a good solubility in water. The additives are components which, when added to 4,4-dimethyl-5α-cholesta-8,14,24-triene-3β-ol provides a composition which can be used to prepare an aqueous solution containing 4,4-dimethyl-5α-cholesta-8,14,24-triene-3β-ol wherein the concentration of MAS is sufficiently high, preferably above about 0.001 µg/ml.

Hence, another embodiment of this invention relates to a closed container having a low content of oxygen and further containing a solid composition with high aqueous solubility comprising 4,4-dimethyl-5α-cholesta-8,14,24-triene-3β-ol and an additive. According to a preferred embodiment, this invention relates to a closed container containing an atmosphere having a low content of oxygen and further containing a solid composition with high aqueous solubility comprising 4,4-dimethyl-5α-cholesta-8,14,24-triene-3β-ol and an additive.

According to a further embodiment, this invention relates to a container, wherein the content of oxygen in the atmosphere is below about 10 %, preferably below about 5 %, more preferred below about 1 % (volume/volume). According to a still further embodiment, this invention relates to a container, wherein a substantial part of the atmosphere is nitrogen or argon. According to a still further embodiment, this invention relates to a container, wherein the content of nitrogen or argon in the atmosphere is above about 90 %, preferably above about 95 %, more preferred above about 99 % (volume/volume).

Examples of preferred additives are water-soluble proteins such as serum albumin, e.g., human serum albumin (hereinafter designated HSA), optionally in recombinant form, enzymes and phosphoglycerides such as phosphatidylethanolamin, phosphatidylcholine, phosphatidylserine, and phosphatidylnositol. As is known by the skilled art worker, albumin can, for example, be prepared from serum or by genetic engineering (recombinant) and, consequently, the products prepared can be designated serum albumin or recombinant albumin, respectively.

According to one embodiment, this invention relates to a container, wherein the additive is a protein or a phosphorglycerid, preferably serum albumin, most preferred human serum albumin, optionally in recombinant form. According to a further embodiment, this invention relates to a container, wherein the additive is serum albumin, optionally in recombinant form. According to a still further embodiment, this invention relates to a container, wherein the additive is human serum albumin, optionally in recombinant form. According to a still further embodiment, this invention relates to a container, wherein the content of additive in the solid compositions is above about 90 %, preferably above about 95 %, even more preferred above about 98 %, and most preferred above about 99 %.

Preferably, the solid compositions have a content of water below about 10 %, preferably below about 5 %, more preferred below about 1 % (weight/weight). According to a preferred embodiment, this invention relates to a container, wherein the content of water in the solid composition is below about 10 %, preferably below about 5 %, more preferred below about 1 % (weight/weight).

Preferably, the solid compositions have a content of organic solvent below about 10 %, preferably below about 5 %, more preferred below about 1 % (weight/weight). According to a preferred embodiment, this invention relates to a container, wherein the content of organic solvent in the solid composition is below about 10 %, preferably below about 5 %, more preferred below about 1 %.

Preferably, the solid compositions have a content of 4,4-dimethyl-5α-cholesta-8,14,24-triene-3β-ol below about 1 %, preferably below about 0.1 %, more preferred below about 0.05 % (weight/weight). According to a preferred embodiment, this invention relates to a container, wherein the content of 4,4-dimethyl-5α-cholesta-8,14,24-triene-3β-ol in the solid composition is below about 10 %, preferably below about 5 %, more preferred below about 2 %, most preferred below about 1 % (weight/weight).

Preferably, the solid compositions have a content of additive above about 50 %, preferably above about 80 %, even more preferred above about 99 %, and most preferred above about 99.9 %.

Preferably the weight ratio between 4,4-dimethyl-5α-cholesta-8,14,24-triene-3β-ol and the additive is in the range from about 1:10, preferably from about 1:50, to about 1:5,000. A preferred range is about 1:1000.

Preferred solid compositions are such which can be treated with an aqueous medium containing no or only low concentrations of organic solvent resulting in a solution having a sufficiently high concentration of 4,4-dimethyl-5α-cholesta-8,14,24-triene-3β-ol e.g., above about 0.001 µg/ml, preferably above about 1 µg/ml. Preferably, these aqueous media contain below about 1 %, preferably below about 0.5 %, more preferred below about 0.1 % of organic solvent (weight/weight). Earlier, several attempts to prepare compositions fulfilling this requirement have failed.

Generally, the solid composition is prepared in a manner known *per se*.

One way of preparing the solid compositions is to prepare a solution of MAS in an organic solvent such as ethanol and, then, to prepare an aqueous solution of the additive. Thereafter, the two solutions are mixed. After mixing the two solutions, the solvent is evaporated or allowed to evaporate. The evaporation can be accelerated by using continuous airflow over the mixed solutions, vacuum drying, freeze drying or any other feasible methods generally known to remove the solvent. Preferably, all these process steps are performed in the absence of oxygen or in the presence of only a minor amount of oxygen. Similarly, the final solid composition is kept in an atmosphere containing no or only a minor amount of oxygen. A preferred way of doing this is to store the solid composition in a closed container wherein the atmosphere has a low content of oxygen, e.g., in nitrogen or argon. These containers may be glass containers or containers of plastic having no undesired action on the solid composition. Preferred examples of such containers are capped vials, capouls, sealed dishes for IVF or IVM treatment or other sealed containers. Another preferred way of avoiding the contact with oxygen is to store the solid composition in a closed container *in vacuo.* For example, the containers can be sealed at the end of the freeze drying procedure.

The containers of this invention are filled with the atmosphere and the solid composition mentioned in the claims below in a manner known *per se*. Alternatively, there is vacuum in the containers of this invention.

The closed container having a low content of oxygen and further containing a solid composition comprising 4,4-dimethyl-5α-cholesta-8,14,24-triene-3β-ol and an additive can be prepared by preparing a solid composition comprising MAS and an additive by freeze drying and by closing the container *in vacuo.*

The closed container containing an atmosphere having a low content of oxygen and further containing a solid composition comprising MAS and an additive can be prepared by preparing a solid composition comprising MAS and an additive, by filling the solid composition in a container, by filling the container with an atmosphere having a low content of oxygen, and by closing the container.

The process of preparing the container of this invention may be performed under conditions where there is a low concentration of oxygen, preferably in an atmosphere having a low content of oxygen. Altgematively, the process may be performned at conditions where the preparation of the solid composition comprising MAS and an additive is performed *in vacuo.*

The product marketed could be a delivery system having one or more depressions or hollows. Hereinafter, these depressions and hollows are mutually designated hollows. At least one of these hollows contains a solid composition and, in the same hollow, the atmosphere has a low content of oxygen, e.g., is nitrogen or argon, or is vacuum. A convenient way of placing the solid 4,4-dimethyl-5α-cholesta-8,14,24-triene-3β-ol therein is first to place a solution containing 4,4-dimethyl-5α-cholesta-8,14,24-triene-3β-ol and the additive in the hollow and thereafter to evaporate the solution, preferably in an atmosphere having a low content of oxygen, e.g., in nitrogen or argon, or in vacuum. In this way, the evaporation residue, i.e., the solid composition, is placed directly in the hollow in said device (delivery system).

According to one embodiment, this invention relates to a container which is a device having one or more hollows among which at least one of the hollows contains an atmosphere with a low content of oxygen and the solid composition. Alternatively, there is vacuum in the container.

Since the solid compositions are to be used for the treatment of oocytes, it is important that the solid compositions do not contain constituents that influence the oocytes negatively.

According to another embodiment, this invention relates to a container containing a solid composition that can be used for preparing an aqueous solution with the characteristics mentioned in any of the following preferred embodiments. According to a preferred embodiment, this invention relates to a container containing a solid composition that can be used for preparing an aqueous solution which when used for the treatment of oocytes can result in a percentage germinal vehicle breakdown (GVB) of at least 50 %, preferably at least 80 %. According to a preferred embodiment, this invention relates to a container containing a solid composition from which, when an aqueous media is added to the solid composition, a solution containing 4,4-dimethyl-5α-cholesta-8,14,24-triene-3β-ol in a concentration of above about 0.001 µg/ml, preferably above about 0.01 µg/ml, more preferred above about 0.1 µg/ml, even more preferred above about 1.0 µg/ml, and most preferred about 10 µg/ml, and even more preferred about 100 µg/ml, can be obtained. According to a preferred embodiment, this invention relates to a container containing a solid composition from which, when an aqueous media is added to the solid composition, a solution containing 4,4-dimethyl-5α-cholesta-8,14,24-triene-3β-ol in a concentration of below about 0.1 g/ml, preferably below about 0.01 g/ml, can be obtained. According to a preferred embodiment, this invention relates to a container containing a solid composition from which, when water is added to the solid composition, an aqueous solution wherein the content of organic solvent is below about 0.1 %, preferably below about 0.05 %, most preferred below about 0.01 %, can be obtained.

One way of using the solid compositions is to dissolve the composition in an aqueous medium such as water and then, if desired, to add other constituents that may have a favourable influence on the maturation of the oocytes.

Another way of using the composition is to dissolve it in a media normally used for IVF or IVM.

The mentioning herein of a reference is no admission that it constitutes prior art.

Herein, the word "comprise" is to be interpreted broadly meaning "include", "contain" or "comprehend" (vide, the EPO guidelines C 4.13).

The present invention is further illustrated by the following examples which, however, are not to be construed as limiting the scope of protection. The features disclosed in the foregoing description and in the following examples may, in any combination thereof, be material for realising the invention in diverse forms thereof.

### Example 1

Method used for determining whether a compound can be used as the additive in the solid compositions or not.

An additive for FF-MAS compositions are characterised by:
Improving the solubility of FF-MAS in ethanol/water (1:2.5 v/v)
Ensuring a clear solution of FF-MAS after reconstitution of the composition in MEM Alpha Medium.
Securing percent GVB is at least 50 % preferable 80 % when tested on oocytes obtained from immature female mice.

Prepare a saturated ethanolic solution of FF-MAS. Blend with an aqueous solution of the additive in the ration 1:2.5. By visual inspection control that surplus FF-MAS is available in the solution. Rotate the solution for 24 hours at room temperature. Filter the solution through 0,22 µm filter, determine the content of FF-MAS by HPLC and calculate the solubility. Transfer 350 µl to 4-well dish and evaporate to dryness at room temperature. Add 500 µl MEM ALPHA medium (Gibcobal). If a clear solution is obtained within half an hour, the composition is tested on oocytes obtained from immature female mice. % GVB obtained is at least 50 %, preferable 80 %, vide example 1.

### Example 2

### Composition containing Human Serum Albumin (HSA).

FF-MAS was dissolved in ethanol (25 µg FF-MAS/ml ethanol). HSA is dissolved in water (1 %). 100 µl of the above mentioned ethanol solution (corresponding to the amount of FF-MAS needed for one treatment) and 350 µl of the above-mentioned HSA solution were mixed in a glass vial. The resulting mixture of FF-MAS and HSA was evaporated to dryness by airflow of argon. After the liquid was evaporated, the vial was sealed by a rubber septum and the vial was placed at a temperature of 2-8°C. At the time for usage, 500 µl of freshly prepared IVM media was added to the residue in the vial and shaken calmly for 1-2 minutes. The IVM media used was TCM 199 with Earle's salts (Sigma) to which was added 0.8% HSA, 2 mM L-glutamine, 0.25 mM sodium pyrovate, 100 IU/ml penicillin G, and 100 microgram/ml streptomycin. The liquid was transferred to a 4-well dish and the composition was tested on oocytes obtained from immature female mice. Percentage GVB obtained, see below in Table 1. The stability of the formulation, as well as the recovery of FF-MAS from the vials were continuously followed, see below in Table 2.

**Table 1: GVB data for FF-MAS in combination with HSA**

| Content of HSA in each vial: 6 mg/ml | | | |
|---|---|---|---|
| **Substance** | **Dose FF-MAS** | **Number of oocytes** | **%GVB** |
| | **(µmol/l)** | | |
| **Control** | - | 35 | 14 |
| **FF-MAS*** | 10 | 35 | 43 |
| **FF-MAS** | 0.24 | 35 | 86 |
| **FF-MAS** | 0.48 | 33 | 88 |
| **FF-MAS** | 1.2 | 35 | 91 |
| **FF-MAS** | 2.4 | 34 | 97 |
| **HSA-control** | - | 33 | 9 |

| | | | |
|---|---|---|---|
| * internal control dissolved in ethanol | | | |

**Table 2: Stability of FF-MAS in combination with HSA**

| Content of FF-MAS in each vial: 10 µg/ml | | | |
|---|---|---|---|
| Content of HSA in each vial: 5 mg/ml | | | |
| **Storage Conditions** | **Months of Storage** | **Assay µg/ml** | **% Recovery** |
| **5°C** | 2 months | 10,15 | 101% |
| | 3 months | 10,3 | 103% |
| **25 °C / 60 %RH** | 2 months | 9,71 | 97% |
| | 3 months | 9,5 | 95% |

| | | | |
|---|---|---|---|
| "RH" designates relative humidity. | | | |

### Example 3

### Preparation:

FF-MAS was dissolved in ethanol (20 µg FF-MAS/ml ethanol) and HSA was dissolved in water (1 %). 100 µl of the above mentioned ethanol solution (corresponding to the amount of FF-MAS needed for one treatment) and 250 µl of the above-mentioned HSA solution were mixed and 350 µl of the combined liquid was aliquoted in a glass vials. The vials were placed at -80°C for one night. All samples were thereafter freeze-dried at -45°C for 16 hours followed by 8 hours freeze drying at -15°C and 8 hours freeze drying at +25°C. Before the freeze dryer was opened all vials were automatically sealed with rubber septums by a pressure of nitrogen. The vials were closed with a capsule and placed at a temperature of 2-8°C. At the time for usage, 500 µl of freshly prepared IVM media was added to the residue in each vial and shaken calmly for 1-2 minutes. The IVM media used was the same as that which was used in example 2. The liquid was transferred to a 4-well dish and the composition was tested on oocytes obtained from immature female mice. Percentage GVB obtained, see below in Table 3. The recovery of FF-MAS from the vials was determined on HPLC to be about 105%, corresponding to 2.10 µg FF-MAS/vial.

**Table 3: GVB data from 2 tests on FF-MAS+HSA compared to internal control**

| **Substance** | **Dose FF-MAS** | **Number of oocytes** | **%GVB** |
|---|---|---|---|
| | **(µmol/l)** | | |
| **FF-MAS*** | 10/10 | 27/41 | 85/83 |
| **FF-MAS + HSA** | 5/5 | 28/108 | 100/95 |

| | | | |
|---|---|---|---|
| *Internal control, FF-MAS dissolved in ethanol | | | |

### Example 4

A comparison on the solubility of FF-MAS in solutions containing either recombinant human albumin (hereinafter designated r-HA) or HSA has been performed. The solutions were prepared as in example 4, with 2.5 mg HSA or r-HA in each vial together with 2 µg FF-MAS. The solutions were filtered through a 0.22 µm filter but not freeze-dried prior to analysis. The recovery data are listed in table 4 below.

**Table 4: Recovery of FF-MAS from solutions prior to freeze-drying.**

| **EtOH:Water** | **FF-MAS + HSA** | **FF-MAS + r-HA** |
|---|---|---|
| 1:2.5 | 62% | 37% |
| 1:5 | 90% | 59% |
| 1:12.5 | 90% | 85% |
| 1:25 | 76% | 66% |
| 1:50 | 81 % | 46% |
| 1:100 | 75% | 24% |

As seen from Table 4, the ideal ratio between ethanol and water is about 1:12.5.

### Example 5

FF-MAS was dissolved in ethanol (77 µg FF-MAS/ml ethanol) and HSA or r-HA was dissolved in water (1 %). 26 ml of the above mentioned ethanol solution and 324 ml of the above-mentioned HSA solution or r-HA solution were mixed and 350 µl of the combined liquid was aliquoted in glass vials (corresponding to the amount of FF-MAS needed for one IVM treatment). The vials were placed at -35°C for one night. All samples were thereafter freeze-dried at -30°C for 16 hours followed by 8 hours freeze drying at -15°C and 8 hours freeze drying at +25°C. Before the freeze dryer was opened all vials were automatically sealed with rubber septums *in vacuo.* The vials were closed with a capsule and stored for stability testing. At the time for usage, 500 µl of freshly prepared IVM media was added to the residue in each vial and shaken calmly for 1-2 minutes. The IVM media used was the same as that which was used in example 2. The results obtained are shown in table 5.

**Table 5**

| | **-18°C** | **+5°C** | **25°C/60% RH** | **40°C** |
|---|---|---|---|---|
| **FF-MAS + HSA** | No significant degradation after 6 months. | No significant degradation after 6 months. | No significant degradation after 6 months. | No significant degradation after 6 months. |
| **FF-MAS + r-HA** | N/A | No significant degradation after 3 months. | N/A | N/A |

| | | | | |
|---|---|---|---|---|
| "RH" designates relative humidity. | | | | |

### Example 6

In order to test the robustness of the formulation, bulk solutions with different ratios between FF-MAS and HSA has been prepared. The recovery of FF-MAS was determined on HPLC on the bulk solutions and none of the samples were freeze dried. The ratio between ethanol and water was kept at 1:12.5 in all samples. Results see Table 6, n=2 in all samples. The analyses were performed right after the preparation.

**Table 6**

| | **FF-MAS /vial** | **HSA /vial** | **Ratio FF-MAS:HSA** | **Content of FF-MAS in bulk** | **Found FF-MAS** | **Recovery** |
|---|---|---|---|---|---|---|
| **I** | 50µg | 2.5 mg | 1:50 | 143µg/ml | 133µg/ml | 93% |
| **II** | 10µ9 | 2.5 mg | 1:250 | 29µg/ml | 27.7µg/ml | 96% |
| **III** | 1µg | 2.5 mg | 1:2500 | 2.9µg/ml | 2.61µg/ml | 90% |
| **IV** | 0.1µg | 2.5 mg | 1:25000 | 0.29µg/ml | 0.29µg/ml | 100% |

During the preparation a slight precipitation was observed in the solution containing the highest amount of FF-MAS (143 µg/ml), but as seen in table 6, the recovery of FF-MAS from all solutions was ≥90%, which is very satisfactory.

### Example 7

Test on mouse oocytes on formulations with FF-MAS and r-HA prepared as described in example 4 and subsequently freeze-dried as described in example 6 has been performed. Five tests were conducted and the results are listed in Table 7.

**Table 7: GVB data from 5 tests on FF-MAS+r-HA compared to internal control**

| **Substance** | **Dose FF-MAS** | **Number of tested oocytes** | **%GVB** |
|---|---|---|---|
| | **(µmol/l)** | | |
| **FF-MAS*** | 10 | 180 | 85 |
| **FF-MAS + r-HA** | 5 | 188 | 87 |

| | | | |
|---|---|---|---|
| *Internal control, FF-MAS dissolved in ethanol | | | |

## Claims

1. A closed container having a content of oxygen which is below about 0.01 mole per litre container volume and further containing 4,4-dimethyl-5α-cholesta-8,14,24-triene-3β-ol,

2. A closed container, according to the preceding claim containing a solid composition with high aqueous solubility comprising 4,4-dimethyl-5α-cholesta-8,14,24-triene-3β-ol and an additive wherein the high aqueous solubility is above about 0.001 µg/ml.

3. A container, according to the preceding claim, **characterised in that** the additive is a protein or a phosphorglycerid, preferably serum albumin, most preferred human serum albumin, optionally in recombinant form.

4. A container, according to the preceding claim, **characterised in that** the additive is serum albumin, optionally in recombinant form.

5. A container, according to the preceding claim, **characterised in that** the additive is human serum albumin, optionally in recombinant form.

## Patentansprüche

1. Geschlossener Behälter mit einem Sauerstoffgehalt, der unter etwa 0,01 mol pro Liter Behältervolumen liegt, und ferner enthaltend 4,4-Dimethyl-5α-cholesta-8,14,24-trien-3 β-ol.

2. Geschlossener Behälter nach dem vorangehenden Anspruch, enthaltend eine feste Zusammensetzung mit einer hohen Löslichkeit in Wasser, umfassend 4,4-Dimethyl-5α-cholesta-8,14,24-trien-3-βol und einen Zusatz, wobei die hohe Löslichkeit in Wasser über etwa 0,001 µg/ml liegt.

3. Behälter nach dem vorangehenden Anspruch, **dadurch gekennzeichnet, dass** der Zusatz ein Protein oder ein Phosphorglycerid, vorzugsweise Serumalbumin, besonders bevorzugt Humanserumalbumin, wahlweise in rekombinanter Form, ist.

4. Behälter nach dem vorangehenden Anspruch, **dadurch gekennzeichnet, dass** der Zusatz Serumalbumin, wahlweise in rekombinanter Form, ist.

5. Behälter nach dem vorangehenden Anspruch, **dadurch gekennzeichnet, dass** der Zusatz Humanserumalbumin, wahlweise in rekombinanter Form, ist.

## Revendications

1. Un récipient fermé ayant un contenu d'oxygène qui est inférieur à environ 0,01 mole par litre de volume de récipient et contenant en outre du 4,4-diméthyl-5α-cholesta-8,14,24-triène-3β-ol,

2. Un récipient fermé selon la revendication précédente, contenant une composition solide de solubilité aqueuse élevée comprenant du 4,4-diméthyl-5α-cholesta-8,14,24-triène-3β-ol et un additif, où la solubilité aqueuse élevée est supérieure à environ 0,001 µg/ml.

3. Un récipient selon la revendication précédente, **caractérisé en ce que** l'additif est une protéine ou un phosphoglycéride, de préférence de la sérum albumine, très préférentiellement de la sérum albumine humaine, éventuellement sous forme recombinante.

4. Un récipient selon la revendication précédente, **caractérisé en ce que** l'additif est la sérum albumine, éventuellement sous forme recombinante.

5. Un récipient selon la revendication précédente, **caractérisé en ce que** l'additif est la sérum albumine humaine, éventuellement sous forme recombinante.
